(19) Europäisches Patentamt
European Patent Office
Office européen des brevets

(11) Publication number: **0 256 611**
**A1**

(12) **EUROPEAN PATENT APPLICATION**

(21) Application number: 87302520.9

(22) Date of filing: 24.03.87

(51) Int.Cl.[3]: A 61 K 9/22
A 61 K 9/00, A 61 K 47/00
//A61K31/71, A61K31/34,
A61K31/415, A61K31/495,
A61K31/57

(30) Priority: 08.08.86 JP 187368/86

(43) Date of publication of application:
24.02.88 Bulletin 88/8

(84) Designated Contracting States:
AT BE CH DE ES FR GB GR IT LI LU NL SE

(71) Applicant: Squibb Japan Inc. Nagai International Building
12-19, Shibuya 2-chome Shibuya-ku
Tokyo(JP)

(72) Inventor: Okada, Yasunobu
11-12-201 Nishi Nakanobu 2-chome
Shinagawa-ku Tokyo(JP)

(72) Inventor: Tanaka, Makoto
2-3-403 Ochiai 4-chome
Tama-shi Tokyo(JP)

(72) Inventor: Ohkubo, Kazue
17-9 Tsijido Motomachi 2-chome
Fugisawa-shi Kanagawa(JP)

(74) Representative: Thomas, Roger Tamlyn et al,
D. Young & Co. 10 Staple Inn
London WC1V 7RD(GB)

(54) **Oral drug delivery systems.**

(57) Oral drug delivery systems are provided formed of compositions which include one or more hydrophilic polymers, such as gelatin, sodium carboxymethyl cellulose, carrageenan or sodium alginate or mixtures thereof; an ointment base such as mineral oil containing dispersed polyethylene (Plastibase); and pullulan as a disintegrating agent for the composition. Such oral drug delivery compositions may be used to deliver to the oral mucosa active ingredients such as steroids, anti-fungal agents, anti-bacterial agents and the like.

EP 0 256 611 A1

# ORAL DRUG DELIVERY SYSTEMS

The present invention relates to an oral drug delivery system which contains one or more hydrophilic polymers, an ointment base, and pullulan as a disintegrating agent, which system when applied to an oral lesion, and upon absorption of water, has high oral tissue affinity so that it can retain active ingredients at the desired site for prolonged periods without causing undue irritation to the oral mucosa.

Drug delivery systems for delivering drugs to the oral mucosa include tinctures, buccal formulations and ointments. Tinctures are easily applied to oral lesions but may be ingested into the body within a relatively short time after application. Buccal formulations may be retained in the oral cavity for longer periods than tinctures; however, they are usually larger in size and thus uncomfortable to retain in the oral cavity and often cause tissue irritation. In addition, they may be separated from lesions by

saliva or other exudate. Conventional ointment formulations may be readily applied. However, in many cases, they also may be readily removed from the oral cavity by mechanical movement of the oral tissue such as during speaking or eating. In addition, conventional ointments may bleed during storage or even after application. Moreover, bleeding of active ingredients from ointments after application to oral lesions may cause active ingredients to be separated from such lesions and carried by saliva to other locations in the oral cavity.

A discussion of prior art patents relating to various drug delivery systems including oral delivery systems and/or oral adhesives follows.

U. S. Patent No. 3,029,187 to Steinhardt discloses anhydrous adhesive pharmaceutical vehicles specifically designed for adhering active ingredients to the oral mucosa which vehicles are formed of an intimate mixture of gelatin and a topically-acceptable vehicle such as petrolatum, lanolin, benzoinated lard, hydrogenated cotton seed oil, carboxymethyl cellulose, pectin, karaya gum, tragacanth, Irish moss extracts, alginates, polyvinyl pyrrolidone, carbo gum, guar gum and pre-treated water-soluble starch. Active ingredients which may be carried by such vehicles include antiseptics, anesthetics, steroids, hormones and antibiotics.

U. S. Patent No. 3,029,188 to Cyr et al discloses gelatin oral adhesive pharmaceutical preparations which include an intimate admixture of particulate gelatin with mineral oil containing

thickening agent, such as polyethylene, dispersed therein. Cyr et al indicate that portions of the gelatin may be substituted by other gums such as carboxymethyl cellulose, pectin, karaya gum, tragacanth, Irish moss extracts, alginates, polyvinyl pyrrolidone, carob gum, guar gum and pre-treated water-soluble starch.

U. S. Patent No. 3,312,594 to Cyr et al discloses a long-lasting troche which contains a medicament and equal portions of pectin, gelatin and carboxymethylcellulose; the troche interacts with saliva to dissolve in the mouth to form an adhesive composition which secures and retains the medicament to the oral mucosa.

U. S. Patent No. 3,984,571 to Chen discloses a liquid carrier for a diagnostic or therapeutic agent which liquid carrier includes a fine particle size hydrocolloid, such as a cellulose ether, suspended in a non-aqueous water-immiscible mobile liquid. When a composition containing the diagnostic or therapeutic agent in the liquid carrier is made to contact a moist surface, the mobile liquid is drained off and the hydrocolloid (carrying the diagnostic or therapeutic agent) attaches itself to the surface.

U. S. Patent No. 4,542,020 to Jackson et al discloses antifungal suppository formulations which are substantially free of water which include an antifungal agent such as nystatin together with a hydrocolloid, such as sodium carboxymethyl cellulose or hydroxypropylmethyl cellulose and a low melting suppository base.

Pullulan is a polysaccharide which is a linear (poly) maltotriose linked through $\alpha$-(1→6) bonds on terminal glycopyranosyl groups of the trisaccharide

$$\rightarrow {}^{6}_{\alpha}\mathrm{Glc} \rightarrow {}^{4}_{\alpha}\mathrm{Glc} \rightarrow {}^{4}_{\alpha}\mathrm{Glc}$$

and has a molecular weight ranging from 30,000 to 700,000. It thus has the structure

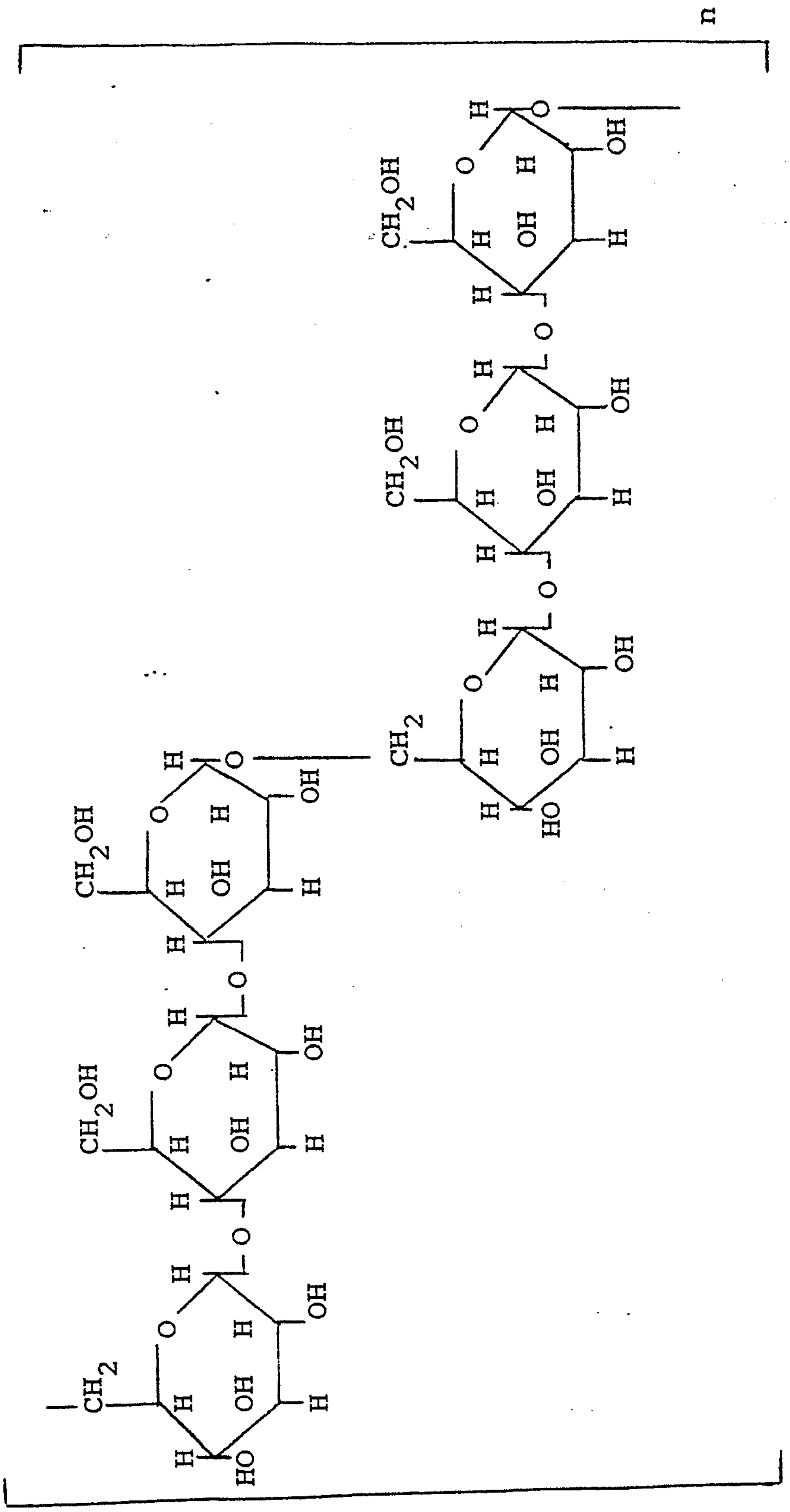

Pullulan

Pullulan has been used in various pharmaceutical preparations including those disclosed in the following patents.

Japanese Patent 61109710 assigned to Taisho Pharmaceut KK discloses suppositories for treatment of hemorrhoids containing polyvinyl alcohol (PVA), pectin, methyl or ethyl cellulose, polyvinyl pyrrolidone, pullulan and/or tragacanth rubber and an oleaginous base, such as cocoa butter, lauric butter, hardened oil, fatty glyceride or water soluble base such as polyethylene glycol.

Japanese Patent No. 61112012 assigned to Gelia Shinyaku Kogy discloses peroral slow-release tablets containing (a) an active ingredient, (b) a mono-, di- or tri-ester of sucrose such as sucrose mono-, di- or tri-palmitate or stearate, and/or an 8-20 C higher fatty acid and (c) a water-soluble high molecular weight substance such as hydroxypropyl cellulose, hydroxypropylmethyl cellulose, polyvinyl pyrrolidone, methyl cellulose, pullulan, dextrin, polyethylene glycol, gum arabic, guar gum or mixtures.

Japanese Patent No. 60123417 assigned to Nitto Electric Ind KK discloses a percutaneous drug delivery device which contains a pressure-sensitive adhesive layer which includes a water-soluble high molecular weight substance formed of a monomer having an ethylenically unsaturated double bond and/or polysaccharides such as polyvinyl alcohol, poly(meth)acrylic acid, sodium poly(methy)acrylate, starch, pullulan, agar-agar or dextrin.

U. S. Patent No. 4,540,602 to Motoyama Shimesu et al discloses a process for preparing a pharmaceutical composition containing a solid drug in the form of finely divided particles no greater than 10 microns in diameter, wherein a solid drug which is substantially water-insoluble is dissolved in a low-boiling hydrophobic organic solvent, the resulting solution is emulsified in water in the presence of a water-soluble high molecular weight substance which is hydroxypropyl cellulose, methyl cellulose, hydroxyethyl cellulose, hydroxypropyl methyl cellulose, hydroxypropyl ethyl cellulose, carboxymethyl cellulose sodium salt, alpha-starch, hydroxypropyl starch, pullulan, gum arabic, tragacanth gum, gelatin, polyvinyl alcohol, polyvinyl pyrrolidone and mixtures thereof and water is thereafter removed.

In accordance with the present invention, a long-lasting bioadhesive oral ointment formulation is provided which upon absorption of water has improved retention of water-soluble or water-insoluble medicament at a desired treatment site in the oral cavity while causing less tissue irritation than prior art formulations. The bioadhesive oral ointment formulation of the invention is formed of a water-soluble or water-insoluble therapeutically active ingredient or medicament, a water-soluble or water-insoluble hydrophilic polymer or hydrocolloid which hydrates, becomes adhesive and increases retention time of the medicament at the

treatment site, pullulan as a disintegrating agent and an ointment base composition.

Thus, in essence, the ointment formulation of the invention is easily applied and soon after application at the desired site in accordance with the aid of the pullulan releases a hydrophilic polymer or hydrocolloid which adheres to the membranes at the desired site and retains a uniform distribution of medicament at the desired site to provide long-lasting treatment.

The pullulan is essential in the ointment formulation of the invention. It acts as a disintegrating agent which controls the disintegration of the ointment formulation to release hydrophilic polymer and active ingredient. The pullulan may be present in an amount within the range of from about 2 to about 27% and preferably from about 16.7 to about 21.6% by weight of the ointment formulation.

The ointment formulation of the invention will also include a water-soluble or water-insoluble medicament in an amount within the range of from about 0.05 to about 25% by weight, and preferably from about 0.05 to about 5% by weight, depending upon the particular medicament employed, a hydrocolloid to impart adhesive qualities, such as gelatin and/or sodium carboxymethyl cellulose, carrageenan or sodium alginate, in an amount within the range of from about 5 to about 60% by weight and preferably from about 15 to about 55% by weight, and more preferably from about 18 to about 44% by weight, and an ointment base in an amount within the range of from about 25 to about 70% by weight and preferably from about 30 to about 65% by

weight, all of the above % being based on the total weight of the ointment formulation.

The medicament which may be employed in the ointment formulation of the invention may be water-soluble or water-insoluble and may include antifungal agents, such as amphotericin B, nystatin, griseofulvin, miconazole, ketoconazole, tioconazole, econazole, clotrimazole, and other macrolide antifungal agents, antibacterials (such as metronidazole, penicillins, ampicillin, neomycin, erythromycin, mupirocin, tyrothricin, gramicidin, cephalosporins, gentamycin and other aminoglycosides, anti-cancer agents (such as 5-fluorouracil), anti-inflammatory agents (such as hydrocortisone, other known steroids such as prednisone, prednisolone, triamcinolone, dexamethasone, and betamethasone), hormones (such as oestriol), analgesic and anti-inflammatory agents such as acetaminophen, phenacetin, aspirin, aminopyrine, sulpyrine, phenylbutazone, mefenamic acid, flufenamic acid, Ibufenac, ibuprofen, indomethacin, colchicine, and Probenecid, and anti-viral agents (such as acyclovir, ribavarin, trifluorothyridine or idoxuridine) antiseptics, hexachlorophene, tetramethyl thiuramdisulfide, benzalkonium chloride, thimerosal, hexylresorcinol, cresols, zinc oxide, methylene blue, boric acid, chloramine-T, gentian violet, phenyl mercuric chloride, phenyl mercuric nitrate basic, acriflavin, sodium perborate, metallic peroxides (e.g. sodium peroxide), sodium permanganate, and the halogens. The medicament will be present in an amount within the range of from about 0.1 to about

25% and preferably from about 0.2 to about 15% by weight depending upon the particular medicament employed and the desired site of action.

Ointment formulations containing such medicaments in accordance with the present invention may be administered up to two times per day or any convenient regimen.

The hydrophilic polymers or hydrocolloids which may be present in the ointment formulation of the invention are water-swellable polymeric substances such as cellulosic polymers and gums. The hydrocolloid will preferably comprise gelatin, cellulose polymers which are cellulose ethers such as methyl cellulose, cellulose alkyl hydroxylates such as hydromethylpropyl cellulose, hydroxypropyl cellulose, hydroxymethyl cellulose or hydroxyethyl cellulose, cellulose alkyl carboxylates such as carboxymethyl cellulose and carboxyethyl cellulose, and alkali metal salts of cellulose alkyl carboxylates, such as sodium carboxymethyl cellulose and sodium carboxyethyl cellulose, or acrylic acid homo- or copolymers or alkali metal salts thereof.

It is to be understood that other known hydrocolloids may be employed in the present invention, including, for example, gum acacia, carrageenan, guar gum, gum tragacanth, gum xanthan, pectin, ammonium or sodium alginate or mixtures thereof.

Preferred hydrocolloids are a mixture of gelatin and sodium carboxymethyl cellulose, carrageenan and/or sodium alginate wherein the gelatin is present in an amount within the range of from

about 2 to about 27% by weight and preferably from about 10 to about 22% by weight of the hydrocolloid mixture and the sodium carboxymethyl cellulose, carrageenan and/or sodium alginate are present in an amount within the range of from about 2 to about 27% by weight and preferably from about 10 to about 22% by weight of the hydrocolloid mixture.

The ointment base suitable for use herein may comprise any conventional ointment formulation suitable for use in the oral cavity, such as disclosed in Remington's "Pharmaceutical Sciences," Sixteenth Edition (Mack Publishing Co., Pa.). Preferred ointment base formulations are set out in U. S. Patent Nos. 3,029,183 and 2,628,187 and comprise mineral oil containing a thickening agent dispersed therein. The thickening agent will be present in an amount of from about 0.25 to about 50% of the combined weight of mineral oil and thickening agent.

The oils which may be used and which are embraced within the term "mineral oil" as used herein are the oils which are liquid at any temperature in the range from 0°C to 60°C and which are essentially hydrocarbons occurring in mineral oil, their distillates and their cracked or polymerized derivatives, an example of the last being polybutene which includes the polymers of butylene and its isomers. The mineral oil may be of any desired character or viscoity, from one which is a thin liquid to one which is so thick that it does not flow at ordinary temperature (20°C).

Thickening (gelling) agents utilizable for dispersion in the mineral oil include, inter alia

paraffin wax, amorphous wax (e.g. microcrystalline wax), ozokerite, animal waxes (e.g. beeswax), vegetable waxes (e.g. castor wax), and hydrocarbon polymers (e.g. polymers of ethylene having an average molecular weight varying from 3,500 to 26,000 and polyisobutylene of a high molecular weight). The preferred thickening agent is polyethylene having a molecular weight of at least 3,500.

A preferred ointment base formulation comprises mineral oil containing polyethylene having a molecular weight of at least 3,500. An example of such an ointment is Plastibase 50W (distributed by E. R. Squibb & Sons, Inc.).

Preferred ointment formulations of the invention are set out below.

| Ingredient | Mg/ointment formulation |
|---|---|
| Medicament | 0.05 to 5 |
| (Triamcinolone acetonide | 0.05 to 5) |
| Pullulan | 16.7 to 21.6 |
| Hydrophilic polymer | 15 to 55 |
| (sodium carboxymethyl cellulose, | |
| carrageenan and/or sodium alginate | (10 to 22 |
| and gelatin) | 10 to 22) |
| Plastibase 50W | 44 to 54 |

The ointment formulation of the invention may be prepared by employing conventional ointment formulating and processing techniques. The active ingredient, pullulan and hydrocolloid are added to the ointment vehicle and the mixture

is mixed according to known procedures to prepare a homogeneous ointment formulation.

The oral formulations of the invention will be in the form of homogeneous pastes and will retain their homogeneity without bleeding upon prolonged storage. Moreover, as will be shown in the Examples, the oral formulations of this invention have excellent oral adhesiveness. Upon application of the oral formulations of this invention to the oral cavity, the formulations are retained for prolonged periods, keeping their original shape at the site of application even after being heated by body temperature, and moistened with saliva. Accordingly, the oral formulations of the invention will maintain its pharmacological efficacy at the site of application without being transferred to other locations in the oral cavity.

The following working Examples represent preferred embodiments of the present invention. Unless otherwise indicated, all temperatures are expressed in degrees Centigrade.

## Examples 1 and 2

Oral steroid ointment formulations in accordance with the present invention having the following composition were prepared as described below.

| Ingredient | Parts by Weight Ex. 1 | Ex. 2 |
|---|---|---|
| Triamcinolone acetonide | 0.1 | 0.1 |
| Hydrocolloid (sodium carboxymethylcellulose and gelatin, 50-50) | 33.4 | 25 |
| Pullulan | 16.7 | 12.5 |
| Plastibase 50W ointment [mineral oil-95% polyethylene (M.W. 21,000 - 5%)] | 49.8 | 62.4 |

The sodium carboxymethyl cellulose, gelatin, pullulan and ointment were kneaded together until a substantially homogeneous ointment was obtained. Thereafter, triamcinolone acetonide was added with thorough mixing until a homogeneous ointment paste was obtained.

Example 3

An oral ointment formulation of the following composition was prepared following the procedure of Example 1 except carrageenan was employed as the hydrocolloid.

| Ingredient | Parts by Weight |
|---|---|
| Triamcinolone acetonide | 0.1 |
| Hydrocolloid (carrageenan and gelatin, 50-50) | 33.4 |
| Pullulan | 16.7 |
| (Plastibase 50W ointment) | 49.8 |

Examples 4 and 5

Oral ointment formulations of the following compositions were prepared following the procedure similar to that described in Example 1 except that sodium alginate was employed as the hydrocolloid.

| Ingredient | Parts by Weight Ex. 4 | Ex. 5 |
|---|---|---|
| Triamcinolone acetonide | 0.1 | 0.1 |
| Hydrocolloid [sodium alginate and gelatin (50-50)] | 33.4 | 25 |
| Pullulan | 16.7 | 12.5 |
| Plastibase 50W ointment | 49.8 | 62.4 |

The adhesiveness and disintegration properties of the oral ointments prepared in the above examples were determined employing the following procedures.

Adhesiveness: A 100 mg sample was placed between two plastic plates of 3 cm in diameter.

The sample was spread to a film of 2 cm in diameter on one plastic plate and then sandwiched between the plastic plates. The two plates were stretched and the critical tearing force at the leaving instance ($g/cm^2$) was determined.

Disintegration: A 200 mg sample (1 x 1 $cm^2$) was placed at the periphery of a plastic plate having a diameter of 12 cm. The plastic plate was dipped into water at 37°C and rotated at 300 rpm in water. The period of time at which 2/5 of the sample remained on the plastic plate was determined.

Results: Following are the results of these samples:

| Ex. No. | Adhesiveness ($g/cm^2$) | Disintegration (min.) |
|---|---|---|
| 1 | 88 | 45 |
| 2 | 68 | 45 |
| 3 | 83 | 40 |
| 4 | 77 | 40 |
| 5 | 67 | 35 |

The above results show that the oral formulations of this invention have good adhesive strength, and high disintegration resistance as seen from the fact that they keep their original shape for a substantial period.

CLAIMS

1. An oral drug delivery system, in the form of an oral ointment formulation, comprising a medicament in an amount within the range of from about 0.05 to about 25% by weight of the total formulation, a hydrocolloid in an amount within the range of from about 0.5 to about 60% by weight of the formulation, pullulan in an amount within the range of from about 0.5 to about 30% by weight of the formulation, and an ointment base therefor, whereupon application of said formulation at the desired site of action in the oral cavity, said ointment formulation with the help of the pullulan and water causes said hydrocolloid and medicament to be released and to adhere to and be retained at the desired site of action.

2. The formulation as defined in Claim 1 wherein the medicament is an antifungal agent.

3. The formulation as defined in Claim 2 wherein the antifungal agent is nystatin, clotrimazole, amphotericin B, miconazole, ketoconazole or griseofulvin.

4. The formulation as defined in Claim 1 wherein the medicament is a steroid.

5. The formulation as defined in Claim 4 wherein said steroid is triamcinolone acetonide.

6. The formulation as defined in Claim 1 wherein said hydrocolloid is gelatin, cellulose ether, a cellulose alkyl hydroxylate, a cellulose alkyl carboxylate, an alkali metal salt of a cellulose alkyl carboxylate, an acrylic acid homo- or copolymer or salt thereof, sodium alginate, carrageenan or mixtures thereof.

7. The formulation as defined in Claim 1 wherein said hydrocolloid is a mixture of gelatin and sodium carboxymethyl cellulose, carrageenan or sodium alginate or mixtures thereof.

8. The formulation as defined in Claim 2 wherein said ointment base is mineral oil containing a thickening agent.

9. The formulation as defined in Claim 1 wherein said ointment base is mineral oil containing polyethylene.

10. The formulation as defined Claim 1 including one or more antifungal agent and/or antibacterial agents.

11. The formulation as defined in Claim 1 wherein the pullulan is present in an amount within the range of from about 2 to about 27% by weight.

12. The formulation as defined in Claim 1 wherein the hydrocolloid is present in an amount within the range of from about 18 to about 44% by weight.

13. The formulation as defined in Claim 12 wherein the hydrocolloid is comprised of from about 2 to about 27% by weight gelatin and from about 2 to about 27% by weight sodium carboxymethyl cellulose, carrageenan or sodium alginate or mixtures thereof, said % being based on the weight of the hydrocolloid mixture.

14. The formulation as defined in Claim 13 wherein the medicament is triamcinolone acetonide, the ointment base is mineral oil thickened with polyethylene and pullulan is present in an amount

within the range of from about 16.7 to about 21.6% by weight of the total formulation.

European Patent Office

# EUROPEAN SEARCH REPORT

0256611
Application number

EP 87302520.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | CHEMICAL ABSTRACTS, vol. 93, no. 5, August 4, 1980, Columbus, Ohio, USA<br>pages 744-745, abstract no. 44 401f<br>& JP-A-80 29 983 (Y. SATO, M. NODA), (03-03-1980)<br>-- | 1-3,6, 7,10-13 | A 61 K 9/22<br>A 61 K 9/00<br>A 61 K 47/00<br>//A 61 K 31/71<br>A 61 K 31/34<br>A 61 K 31/415<br>A 61 K 31/495<br>A 61 K 31/57 |
| Y | CH - A5 - 651 472 (LIFE SAVERS, INC.)<br>* Claims 1,3,4,8; example 1; page 5, left column, lines 46-55 *<br>-- | 1-3,6, 7,10-13 | |
| D,Y | PATENT ABSTRACTS OF JAPAN, unexamined applications, C section, vol. 9, no. 278, November 6, 1985<br>THE PATENT OFFICE JAPANESE GOVERNMENT<br>page 23 C 312<br>* Kokai-no. 60-123 417 (NITTO DENKI KOGYO K.K.) *<br>-- | 1-4,8-10,14 | **TECHNICAL FIELDS SEARCHED (Int Cl.4)**<br>A 61 K 9/00<br>A 61 K 47/00<br>A 23 G 3/00 |
| Y | US - A - 4 059 686 (W. TANAKA et al.)<br>* Claims 1,4-7; column 3, lines 20-34 *<br>-- | 1-4,8-10,14 | |
| Y | EP - A1 - 0 107 941 (TAKEDA CHEMICAL INDUSTRIES, LTD.)<br>* Abstract; claims 1,3-5,7,8; page 9, line 14 -page 10, line 15, example 6; page 3, lines 6,7 in connection with page 5, lines 16-21 *<br>-- | 1-7, 10,11 | |

The present search report has been drawn up for all claims

| Place of search | Date of completion of the search | Examiner |
|---|---|---|
| VIENNA | 13-11-1987 | MAZZUCCO |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

0256611

Application number

European Patent Office

# EUROPEAN SEARCH REPORT

EP 87302520.9

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int Cl 4) |
| Y | FR - A - 2 147 112 (HAYASHIBARA BIOCHEMICAL LABORATORIES, INC.)<br>* Claims 1,4-6,9; example 5 *<br>-- | 1-7,10, 11 | |
| A | AU - B1 - 40 452/78 (ASAHI KASEI KOGYO KABUSHIKI KAISHA)<br>* Claims 1-3; pages 2-4,39,40 *<br>-- | 1,2 | |
| D,A | US - A - 4 540 602 (S. MOTOYAMA et al.)<br>* Claims 1,4,6,10; example 17 *<br>-- | 1-4,6, 10 | |
| D,A | US - A - 3 029 188 (G.N. CYR, H. B. BERNSTEIN)<br>* Claims 1,2,5,9; examples 6,9; column 2, line 69 - column 3, line 5 *<br>---- | 1-4,6-10,12, 13 | TECHNICAL FIELDS SEARCHED (Int Cl 4) |

The present search report has been drawn up for all claims

| Place of search | Date of completion of the search | Examiner |
|---|---|---|
| VIENNA | 13-11-1987 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document